# EUROPÄISCHE PATENTANMELDUNG

(11) **EP 3 960 063 A1**
(43) Veröffentlichungstag der Anmeldung: **02.03.2022**
(21) Anmeldenummer: 21192993.0
(22) Anmeldetag: 25.08.2021
(51) Int. Cl.: A61B 1/00, A61B 1/267

(54) **OPTISCHES INSTRUMENT UND VERFAHREN ZUM HERSTELLEN EINES OPTISCHEN INSTRUMENTS**

(30) Priorität: 01.09.2020 DE 102020122846
(71) Anmelder: Karl Storz SE & Co. KG, 78532 Tuttlingen (DE)
(72) Erfinder: Huber, Christian, 8200 Schaffhausen (CH); Hüls, Dieter, 78333 Stockach (DE); Sterk, Mario, 78194 Immendingen (DE)

(57) **Zusammenfassung**

Ein optisches Instrument zur Betrachtung von Lebewesen zu medizinischen Zwecken umfasst einen Instrumentenkörper (20, 30, 40, 50, 60), wobei in dem Instrumentenkörper (20, 30, 40, 50, 60) wenigstens ein optisches Element (21, 31, 41, 51, 61) zur Aufnahme von Licht aus einem Bereich außerhalb des Instrumentenkörpers (20, 30, 40, 50, 60) und wenigstens eine Beleuchtungseinheit (22, 23, 32, 42, 52a, 52b, 62) zur Beleuchtung dieses Bereichs angeordnet sind. Das optische Element (21, 31, 41, 51, 61) wird von einer Fassung (25, 33, 43, 53, 63) in dem Instrument gehalten, deren distaler Rand sich über das optische Element (21, 31, 41, 51, 61) hinaus erstreckt und in wenigstens einem Abschnitt umgeformt ist, um das optische Element (21, 31, 41, 51, 61) zu halten. Nicht umgeformte Abschnitte (27, 35, 45, 55a, 55b, 65) des distalen Randes dienen als Streulichtschutz, der Beleuchtungslicht von dem optischen Element (21, 31, 41, 51, 61) fernhält. Es wird auch ein Verfahren (70) zum Herstellen eines solchen Instruments vorgeschlagen.

## Beschreibung

Die vorliegende Erfindung betrifft ein optisches Instrument zur Betrachtung von Lebewesens zu medizinischen Zwecken.

Solche Instrumente sind häufig als Endoskope zum Einblick in den Körper, als Mikroskope oder Exoskope zur Betrachtung des Körpers von außerhalb des Körpers oder als Laryngoskope zum Einsatz bei der Intubation ausgebildet.

Endoskope werden heute für vielerlei Anwendungen in Medizin und Technik verwendet. Ein Endoskop umfasst typischerweise einen starren, halbstarren oder flexiblen lang erstreckten Schaft, der zur Einführung in einen Hohlraum geeignet ist. Im distalen (d.h. beobachterfernen) Endbereich des Schafts ist zur Erzeugung eines Bildes eines Objektfelds in dem Hohlraum meist ein Endoskopobjektiv angeordnet. Das endoskopische Bild kann über im Innern des Schafts angeordnete optische oder elektronische Mittel zum proximalen (beobachternahen) Ende des Endoskops weitergeleitet werden, wo es zur Betrachtung bzw. Anzeige für einen Benutzer zur Verfügung steht.

Da in dem beobachteten Hohlraum in der Regel nicht ausreichend Licht vorhanden ist, ist innerhalb des Schafts ferner häufig ein Beleuchtungslichtleiter angeordnet, um Beleuchtungslicht an das distale Ende des Endoskops zu transportieren, wo es zur Beleuchtung des Hohlraums genutzt wird. Zur Einleitung des Beleuchtungslichts in das Endoskop ist in der Nähe des proximalen Endes oft ein Anschluss zum Anschließen eines Lichtleitkabels vorhanden, mit dem das Beleuchtungslicht von einer separaten Lichtquelle zugeführt werden kann.

Alternativ oder zusätzlich kann Licht auch über distal am Schaft angeordnete Beleuchtungsmittel wie LEDs oder Laserdioden bereitgestellt werden.

Bei modernen Videoendoskopen sind am distalen Ende häufig ein oder mehrere Bildsensoren zur Aufnahme eines vom Objektiv erzeugten Bilds angeordnet. Alternativ können die Bildsensoren auch am proximalen Ende des Endoskops angeordnet sein, beispielsweise in einer Handhabe oder in einem Kamerakopf.

Laryngoskope können ähnlich aufgebaut sein und weisen statt eines Schafts meist einen Spatel und einen Handgriff auf. Das Objektiv oder auch der Bildsensor zur Aufnahme von Bildern der Larynx ist meist am Spatel angebracht, ebenso wie die Beleuchtungsmittel. Laryngoskope werden zur sicheren Einführung von Intubationstuben in die Luftröhre von Patienten verwendet.

Objektive und Bildsensoren sind am distalen Ende meist von einem Fenster, einer Linse oder einem anderen optischen Element verschlossen, um die Optik zu schützen und die Instrumente dicht ausgestalten zu können, so dass sie sich für eine intensive Reinigung mit Chemikalien, hohen Temperaturen, Dampf und hohen Drücken eignen.

Mikroskope und Exoskope dienen der Betrachtung des Körpers von außen und stellen dabei ebenfalls optische Elemente und Kameras bereit, ebenso wie Beleuchtungsmittel. Sie sind häufig an Stativen oder Haltearmen befestigt und erlauben die Betrachtung der äußeren Oberfläche des Körpers oder der inneren Organe durch eine geeignete natürliche oder künstliche Öffnung. Häufig sind Mikroskope und Exoskope in der Lage, über geeignete Optiken und digitale Zoomfunktionen ein stark vergrößertes Bild des beobachteten Bereichs zur Verfügung zu stellen.

Allen Instrumenten ist gemein, dass in einem distalen Bereich der Instrumente, d.h. an einem Ende, das dem Patienten zugewandt ist, sowohl optische Mittel zur Aufnahme von Licht als auch Beleuchtungsmittel vorgesehen werden müssen. Aufgrund der räumlichen Nähe dieser Komponenten zueinander besteht häufig das Problem, dass unerwünschterweise Licht aus einem Beleuchtungsmittel austritt und dann direkt oder durch andere Teile des Instruments gestreut in die Optik des Instruments eintritt. Dies verschlechtert die Bildqualität, da dieses Streulicht das eigentlich zur Bilderzeugung verwendete reflektierte Licht aus dem zu beobachteten Bereich überstrahlt und das erzeugte Bild beeinträchtigt. Je kleiner der betreffende Bereich des Instruments ist, in dem optische Mittel und Beleuchtungseinheiten nahe beieinander angeordnet sind, desto schwieriger ist es, das Streulicht von der Optik des Instruments fernzuhalten. Insbesondere im Bereich der Endoskope und Laryngoskope stehen am distalen Ende häufig nur wenige Millimeter Endfläche zur Verfügung, in der sämtliche Komponenten angeordnet sind, teils mit zusätzlichen Arbeitskanaleingängen oder Mitteln zum Spülen und Saugen, die ebenfalls Raum einnehmen.

Aus der DE 10 2013 007 491 A1 ist eine Streulichtblende für medizinische optische Instrumente bekannt, die außen auf den Schaft des Instruments aufgesetzt ist. Diese Blende ist geeignet, Streulicht von Lichtquellen, die sich abseits des distalen Instrumentenendes befinden, von der Optik des Instruments fernzuhalten. Die Blende nimmt jedoch sehr viel Platz ein, ändert die Abmessungen des Instruments und ist nicht geeignet, nahe beieinander angeordnete Optiken und Beleuchtungsmittel voneinander zu trennen.

Die EP 2 491 848 A1 beschreibt ein Videoendoskop mit einem distalen Ende, in das Fenster für Bildgebung und Beleuchtung integriert sind. Die Fenster für die Beleuchtung sind in Vertiefungen angeordnet, während das Fenster für die Bildgebung in einem Vorsprung mit schräger Kante gefasst ist. Nachteilig ist, dass die distale Endfläche für diese Anordnung sehr aufwändig gestaltet sein muss.

In der US 2011 / 0 077 465 A1 sind verschiedene Anordnungen von Lichtquelle und Optik für die Bildaufnahme am distalen Ende eines Videoendoskops dargestellt.

Die CN 2 03 914 844 U beschreibt eine Anordnung zur Verhinderung von Streulicht an einem Endoskop, bei der die Optik und die Beleuchtungseinheiten mit einem speziell ausgestalteten Deckglas abgedeckt sind.

US 2008 / 0 208 006 A1 zeigt ein Videolaryngoskop, bei dem ein distales optisches Element rundherum von einer Fassung umgeben ist. Daneben angeordnete LEDs sind gegenüber der vordersten optischen Fläche zurückgesetzt.

EP 3 613 326 A1 offenbart ein Videoendoskop, das distal mit einem Fenster abgedeckt ist. In das Fenster sind seitlich zwei Vorsprünge nach proximal zwischen der Optik des Instruments und den Beleuchtungseinheiten integriert, um Streulicht fernzuhalten.

In der JP 2004 - 016 410 A ist beschrieben, das distale Ende eines Endoskops mit einer dreiteiligen Linse abzudecken, wobei der mittlere Teil die Optik und die seitlichen Teile die Beleuchtung bedecken. Zwischen den Teilen der Linse sind Schutzplatten eingeklebt, die ein Eindringen von Streulicht in die Optik verhindern.

Die EP 3 613 332 A1 beschreibt einen distalen Teil eines Endoskops mit einer Kamera und einem Beleuchtungsmittel, wobei ein Streulichtschutz in der distalen Endfläche zwischen dem Beleuchtungsmittel und der Kamera angeordnet ist.

Nachteilig an diesen aus dem Stand der Technik bekannten Lösungen ist, dass es sich jeweils um aufwändig zu fertigende Lösungen zur Abblendung von Streulicht handelt, die außerdem zusätzlichen Platz benötigen.

Es besteht daher die Aufgabe, ein Instrument mit einem Streulichtschutz zwischen einem Beleuchtungsmittel und einem optischen Element des Instruments bereitzustellen, der auf kleiner Fläche realisiert werden kann und der selbst die Bildgebung nicht beeinträchtigt. Eine weitere Aufgabe der Erfindung ist die Bereitstellung eines Verfahrens, um ein Instrument mit einem solchen Streulichtschutz möglichst einfach herzustellen.

Diese Aufgaben werden durch eine Vorrichtung nach Anspruch 1 und ein Verfahren nach Anspruch 10 gelöst.

Vorteilhafte Weiterbildungen der Erfindung ergeben sich aus den Unteransprüchen.

Ein optisches Instrument, wie es hier beschrieben ist, ist zur Betrachtung eines Lebewesens zu medizinischen Zwecken geeignet. Es kann sich beispielsweise um ein Endoskop, ein Mikros- / Exoskop oder ein Laryngoskop handeln, wie es zur Diagnose und Behandlung bei Menschen und Tieren eingesetzt wird.

Das Instrument weist einen Instrumentenkörper auf, wobei in dem Instrumentenkörper wenigstens ein optisches Element zur Aufnahme von Licht aus einem Bereich außerhalb des Instrumentenkörpers und wenigstens eine Beleuchtungseinheit zur Beleuchtung dieses Bereichs angeordnet sind. Das optische Element wird von einer Fassung in dem Instrument gehalten, die sich in distaler Richtung über das optische Element hinaus erstreckt und dort einen distalen Rand bildet. Der distale Rand ist in wenigstens einem Abschnitt so umgeformt, dass der umgeformte Abschnitt des distalen Randes das optische Element in distaler Richtung formschlüssig hält. Die Form des distalen Randes in diesem Abschnitt oder in diesen Abschnitten ist also so ausgebildet, dass der distale Rand das optische Element in distaler Richtung formschlüssig hält. Wenigstens ein nicht umgeformter Abschnitt des distalen Randes ist so angeordnet, dass er einen Streulichtschutz bildet, der aus der Beleuchtungseinheit austretendes Beleuchtungslicht von dem optischen Element fernhält. Ein nicht umgeformter Abschnitt des distalen Randes ist vorliegend ein Abschnitt des distalen Randes, der nicht dazu beiträgt oder nicht derart ausgebildet ist, das optische Element zu halten und zu sichern. Die Form des distalen Randes im Bereich des nicht umgeformten Abschnitts oder der nicht umgeformten Abschnitte ist also derart ausgebildet, dass sie das optische Element in distaler Richtung nicht durch Formschluss hält.

Das Instrument kann im Instrumentenkörper oder in einer separaten Kamera außerdem einen oder mehrere Bildsensoren zur Aufnahme von Bildern und Video aufweisen. Das optische Element ist beispielsweise Teil eines Objektivs zur Bildgebung, ein Prisma oder auch ein Fenster oder Deckglas, z.B. in Art einer planparallelen Platte. Es kann rund und beispielsweise aus Glas oder Kunststoff gefertigt sein. Das optische Element ist von einer Fassung gehalten, beispielsweise in Form eines Zylinders mit einem kreisförmigen Querschnitt, der Teil des Instruments ist oder im Instrument angeordnet ist. Die Fassung kann aus Metall sein. Die Fassung kann das optische Element unmittelbar umgeben, so dass der distale Rand direkt an das optische Element angrenzt. Ein umgeformter Abschnitt des distalen Randes kann ein, insbesondere flacher, Teil des distalen Randes sein, der sich in einer Richtung senkrecht oder näherungsweise senkrecht zur Längsachse der Fassung und parallel zum optischen Element erstreckt. Dabei wird das optische Element an seiner distalen Fläche in seinem äußeren Randbereich teilweise von dem umgeformten Abschnitt des distalen Randes der Fassung überdeckt und gehalten. Ein nicht umgeformter Abschnitt des distalen Randes kann ein im Wesentlichen gerader Teil des distalen Randes sein, der sich in Erstreckungsrichtung der Fassung nach distal über das optische Element hinaus erstreckt und dort einen Streulichtschutz ausbildet.

Üblicherweise werden solche optischen Elemente geklemmt, geklebt oder ihr Rand am Umfang metallisiert und das Element in die Fassung eingelötet. Da medizinische Instrumente einer intensiven Reinigung in Reinigungsflüssigkeit oder Autoklaven zugeführt werden müssen, müssen diese nach außen abgedichtet gefertigt werden. Leider ist es ein häufiges Problem, dass durch unsachgemäße Handhabung oder Reinigung oder durch andere Umstände beispielsweise ein Klebstoff, der das optische Element in der Fassung hält, ausgewaschen wird. Der Klebstoff wird also unbeabsichtigt mit entfernt und das optische Element kann dadurch während der Verwendung des Instruments aus der Fassung fallen. Daher ist es ein Merkmal des vorliegend beschriebenen Instruments, dass die Fassung so ausgebildet und das optische Element so in der Fassung angeordnet ist, dass die Fassung nach distal, also aus dem Instrument heraus und in Richtung des Patienten, vorsteht und dort um das Element herum einen distalen Rand bildet. Der distale Rand wird bzw. ist in wenigstens einem Teilabschnitt des distalen Randes umgeformt, so dass er das optische Element teilweise formschlüssig umgreift und dieses zusätzlich nach distal sichert und hält. Mit Abschnitt ist vorliegend ein Abschnitt des Umfangs des distalen Randes gemeint. Auf diese Weise kann das Element auch dann nicht aus der Fassung fallen, wenn ein Klebstoff oder dergleichen das Element nicht mehr befestigt.

Die eine oder mehreren Beleuchtungseinheiten sind zusammen mit dem optischen Element in dem Instrument angeordnet und beleuchten beispielsweise einen Operationssitus, also einen Raumbereich distal vor dem Instrument, insbesondere den Bereich, aus dem von dem optischen Element Licht erfasst und einer Bildgebung zugeführt wird. Die Beleuchtungseinheit kann eine LED (Light Emitting Diode), eine Laserdiode oder ein klassisches Leuchtmittel wie eine Glühbirne sein, sie kann aber auch aus den Faserenden einer Glasfaserbeleuchtung bestehen, deren angeschlossene Lichtquelle sich an anderer Stelle im oder außerhalb des Instruments befindet. Die Beleuchtung kann einfarbig, mehrfarbig oder weiß sein und kann beispielsweise geeignet sein, eine Fluoreszenz anzuregen. Es ist auch bekannt, weißes Licht durch Anregung eines im Instrument angeordneten sogenannten Phosphors mit einem Laser zu erzeugen.

LEDs sind kleine, aber flächige Beleuchtungsmittel, die häufig hinter einem Fenster und einer transparenten Vergussmasse an medizinischen Instrumenten angebracht sind. Hierbei kann es sein, dass nicht nur die LED-Fläche sondern auch die Fläche der Vergussmasse Licht streut und abgibt. Wenn vorliegend von einer Beleuchtungseinheit die Rede ist, kann damit auch diese ganze lichtabstrahlende Fläche gemeint sein. Manchmal ist die Fläche der LED auch gleich der lichtabstrahlenden Fläche. Gegebenenfalls kann ein optisches Element wie eine Linse Teil der Beleuchtungseinheit sein, die das Beleuchtungslicht formt und hilft, bestimmte Bereiche auszuleuchten.

An medizinischen Instrumenten steht insbesondere in der minimalinvasiven Medizintechnik meist nur sehr wenig Platz für Beleuchtung, Optik und weitere Komponenten zur Verfügung, häufig nur wenige Millimeter. Auch kleine Beleuchtungseinheiten wie LEDs können eine große Beleuchtungsfläche relativ zu ihrer Dicke oder Höhe, also ihrem Raumbedarf in distaler Richtung von der Oberfläche weg, haben. Gleichzeitig sollen Beleuchtung und Optik bzw. Bildgebungsmittel in der Instrumentenfläche in etwa in einer Ebene angeordnet sein, um sich nicht gegenseitig zu blockieren und abzuschatten. Beispielsweise sind das optische Element und die Beleuchtungseinheit oder die Beleuchtungseinheiten in einer distalen Endfläche des Instruments angeordnet. Das optische Element ist das am distalsten gelegene optische Element des Instruments. Es ist daher leicht möglich, dass direktes Licht oder Streulicht aus den Beleuchtungseinheiten in die Optik fällt und dadurch die Bildqualität negativ beeinträchtigt. Wenn in diesem Text von Streulicht die Rede ist, soll damit auch direkt von der Beleuchtungseinheit einfallendes Licht gemeint sein und umgekehrt.

Beim vorliegenden Instrument ist nun festgestellt worden, dass der Einfall von Streulicht erheblich reduziert werden kann, wenn der distale Rand der Fassung nicht vollständig zum Halten des optischen Elements umgeformt wird. Stattdessen wird der distale Rand nur in wenigstens einem Abschnitt umgeformt, der ausreichend ist, das Element zu halten. Ansonsten bleibt der distale Rand als Blende bzw. Streulichtschutz stehen. Dabei wird der Abschnitt des distale Randes, der umgeformt wird, so geeignet gewählt, dass der nicht umgeformte Teil als Streulichtschutz geeignet ist. Dies wird im Folgenden noch genauer beschrieben.

Um das optische Element nicht in seiner Funktion zu beeinträchtigen, sollte der umgeformte Abschnitt des distalen Randes möglichst klein sein. Gleichzeitig sollte eine Fertigung des Instruments erleichtert werden. Das Umformen kann daher beispielsweise durch Umbiegen erfolgen. Bei einem Werkstoff wie Kunststoff kann der Rand auch warm bzw. thermoplastisch verformt werden.

Insbesondere wird vorliegend vorgeschlagen, die Fassung aus Metall zu bilden. Weiter wird vorgeschlagen, den oder die Abschnitte des distalen Randes durch Verstemmen mechanisch umzuformen. Dies erfolgt beispielsweise durch ein passendes Stempelwerkzeug. Der distale Rand kann dabei kalt oder heiß verstemmt werden. Das Verstemmen ermöglicht es, auch sehr kleine Ränder mit Abmessungen deutlich unter einem Millimeter sicher zu verformen. Das optische Element wird dann durch den verstemmten Abschnitt oder die verstemmten Abschnitte des distalen Randes in der Fassung gehalten.

Damit der verbleibende, nicht umgeformte Teil des distalen Randes einen ausreichenden Streulichtschutz bildet und die Optik nicht beeinträchtigt wird, beträgt die Höhe des distalen Randes der Fassung, die über das optische Element distal hinaussteht, vorzugsweise wenigstens 0,5% und höchstens 5% des Umfangs des distalen Randes. Insbesondere bei minimalinvasiven Instrumenten mit kleinem Durchmesser kann dies bedeuten, dass der nicht umgeformte oder verstemmte distale Rand sehr geringe Abmessungen aufweist. Wenn die Fassung beispielsweise einen Durchmesser von 1 Millimeter aufweist, so ist der distale Rand in dem oder den nicht umgeformten Abschnitten vorzugsweise etwa 0,016 bis 0,16 mm hoch. Da dies auch der Höhe des distalen Randes vor dem Umformen entspricht, ist das Verstemmen als Umformungsmethode für diese kleinen Abmessungen besonders geeignet.

Alternativ ist vorstellbar, dass die Höhe des distalen Randes in dem oder den nicht umgeformten Abschnitten bis zu 10% des Umfangs des distalen Randes der Fassung beträgt.

Die Beleuchtungseinheit weist ferner eine lichtabstrahlende Fläche auf. Der wenigstens eine nicht umgeformte Abschnitt wird vorteilhafterweise so angeordnet, dass er sich zwischen dem optischen Element und der lichtabstrahlenden Fläche befindet. Auf diese Weise wird eine direkte Linie zwischen Beleuchtungseinheit und optischem Element von dem nicht umgeformten Abschnitt unterbrochen und die Optik vor Streulicht geschützt.

Insbesondere ist vorgesehen, dass der wenigstens eine nicht umgeformte Abschnitt des distalen Randes mindestens einen solchen Teil A des Umfangs des distalen Randes umfasst, der einem Winkel αₘₐₓ entspricht, der von Geraden zwischen dem Mittelpunkt des optischen Elements und Punkten der Begrenzung der lichtabstrahlenden Fläche derart aufgespannt wird, dass der Winkel maximal wird.

Wenn man von einem Mittelpunkt des optischen Elements aus gedanklich gerade Linien zu der äußeren Begrenzung der lichtabstrahlenden Fläche der Beleuchtungseinheit zieht, so bilden diese Geraden unterschiedliche Winkel miteinander. Betrachtet man den größten Winkel, der durch diese Geraden gebildet werden kann, schließt dieser schließlich die Beleuchtungsfläche, also die lichtabstrahlende Fläche, ein und die Geraden treffen die äußersten Begrenzungen der lichtabstrahlenden Fläche. Innerhalb dieses Winkels verläuft ein Teil A des distalen Randes zwischen den Geraden auf einem Teil seines Umfangs um das optische Element herum. Dieser Teil bildet vorliegend mindestens oder genau den nicht umgeformten Abschnitt des distalen Randes, während der distale Rand darüber hinaus umgeformt ist, beispielsweise durch Verstemmen. Auf diese Weise wird der Streulichtschutz genau zwischen dem optischen Element und der leuchtenden Fläche der Beleuchtungseinheit errichtet.

Weiterhin kann vorgesehen sein, dass der nicht umgeformte Abschnitt des distalen Randes wenigstens einen Teil B des Umfangs des distalen Randes zwischen zwei Punkten a und b umfasst, derart, dass die jeweilige Tangente, die in diesen Punkten den distalen Rand beführt, die lichtabstrahlende Fläche schneidet und die Länge des Teils B zwischen a und b maximal wird.

Der Teil B verläuft also zwischen zwei Punkten a und b auf dem distalen Rand, in denen eine gedachte Tangente an den distalen Rand jeweils gerade noch die lichtabstrahlende Fläche der Beleuchtungseinheit schneidet oder berührt. Dies wird an den äußersten Grenzen der lichtabstrahlenden Fläche der Fall sein und die Punkte a und b liegen bei dieser Überlegung maximal weit auseinander, so dass sie noch von Beleuchtungslicht getroffen werden könnten. Daher kann der nicht umgeformte Abschnitt des distalen Randes wenigstens diesen Teil B umfassen, um besonders gut vor direktem oder Streulicht zu schützen.

Alternativ kann der nicht umgeformte Abschnitt des distalen Randes zwischen diesen Werten liegen, also mindestens den Teil A und höchstens den Teil B nach den oben genannten Definitionen umfassen. Der nicht umgeformte Abschnitt des distalen Randes umfasst also mindestens Teil A und höchstens Teil B des Umfangs zwischen zwei Punkten a und b, derart, dass die jeweilige Tangente, die in diesen Punkten den distalen Rand berührt, die lichtabstrahlende Fläche schneidet oder berührt, so dass die Länge des Teils B maximal wird. Welche Ausgestaltung am geeignetsten ist, wird der Fachmann anhand des konkreten Instruments und beispielsweise der Helligkeit der Beleuchtungsmittel, Eigenschaften des optischen Elements und dergleichen ermitteln.

Das hier beschriebene Instrument ist insbesondere ein Laryngoskop mit einem Handgriff und einem Spatel, wobei das optische Element und die Beleuchtungseinheit am Spatel angeordnet sind. Spatel und Handgriff bilden den Instrumentenkörper. Laryngoskope sind Instrumente zur Visualisierung der Larynx beim Einführen eines Intubationstubus in die Luftröhre. Der Spatel dient als Hebel, um den Mund des Patienten zu öffnen und die Zunge herunterzudrücken und den Tubus einführen zu können. Die Visualisierung erfolgt über das optische Element und die Beleuchtungseinheit am Spatel, beispielsweise ein Objektiv mit Bildsensor, das zusammen mit einer oder mehrerer LEDs am Spatel angeordnet sein kann. Das vom Bildsensor erfasste Bild wird an einem Monitor dargestellt. Ein solches Videolaryngoskop muss nach jeder Anwendung gereinigt werden, weshalb eine sichere Fixierung des optischen Elements sehr wichtig ist. Gleichzeitig darf die Bildgebung nicht durch Streulicht beeinträchtigt werden, da die Intubation zügig und richtig erfolgen muss, um den Patienten nicht zu gefährden.

In einer Ausführungsform des Instruments ist vorgesehen, dass das Instrument wenigstens zwei Beleuchtungseinheiten aufweist und der distale Rand in wenigstens zwei separaten Abschnitten umgeformt ist, so dass die nicht umgeformten Abschnitte Beleuchtungslicht aus beiden Beleuchtungseinheiten von dem optischen Element fernhalten.

Häufig ist zur gleichmäßigen Ausleuchtung eines durch das Instrument betrachteten Bereichs eine Mehrzahl von Beleuchtungseinheiten notwendig, beispielsweise zwei. Diese können beidseitig des optischen Elements angeordnet sein. Der distale Rand der Fassung ist dann so in zwei Abschnitten ausgebildet und umgeformt, dass diese Abschnitte das optische Element halten, während die verbleibenden Abschnitte des distalen Rands als Streulichtschutz dienen und passend zu beiden Beleuchtungseinheiten ausgerichtet sind. Hierfür können die zuvor genannten Merkmale alle analog angewandt werden, um ein besonders vorteilhaftes Instrument auszubilden. Insbesondere kann auch hier der Rand zur Umformung verstemmt worden sein.

Ein Verfahren zum Herstellen eines optischen Instruments zur Betrachtung eines Lebewesens zu medizinischen Zwecken umfasst die Schritte:
- Bereitstellen eines Instrumentenkörpers,
- Anordnen wenigstens eines optischen Elements zur Aufnahme von Licht aus einem Bereich außerhalb des Instrumentenkörpers und wenigstens einer Beleuchtungseinheit zur Beleuchtung dieses Bereichs in dem Instrumentenkörper,
- Anordnen des optischen Elements in einer Fassung, so dass die Fassung sich in distaler Richtung über das optische Element hinaus erstreckt und dort und einen distalen Rand bildet,
- Befestigen der Fassung in dem Instrumentenkörper,
   und
- Umformen des distalen Rands in wenigstens einem Abschnitt, so dass der umgeformte Abschnitt des distalen Randes das optische Element in distaler Richtung formschlüssig hält, und der wenigstens eine nicht umgeformte Abschnitt des distalen Randes so angeordnet ist, dass er einen Streulichtschutz bildet, der aus der Beleuchtungseinheit austretendes Beleuchtungslicht von dem optischen Element fernhält.

Für den Fall, dass das Instrument mehr als eine Beleuchtungseinheit aufweisen soll, kann das Verfahren weiterhin die folgenden Schritte umfassen:
- Anordnen wenigstens einer zweiten Beleuchtungseinheit in dem Instrumentenkörper und
- Umformen eines zweiten separaten Abschnitts des distalen Randes, so dass die nicht umgeformten Abschnitte Beleuchtungslicht aus beiden Beleuchtungseinheiten von dem optischen Element fernhalten.

Der Schritt des Umformens kann insbesondere ein Verstemmen von Abschnitten des distalen Randes umfassen.

Die Schritte des genannten Verfahrens können in anderer Reihenfolge erfolgen, als hier angegeben und es können weitere Herstellungsschritte vorgesehen sein, die nicht Teil der vorliegenden Erfindung sind. Insbesondere kann die Fassung im Bereich des distalen Randes zunächst von einer dafür eingerichteten Vorrichtung in einem oder mehreren Abschnitten umgeformt, insbesondere verstemmt werden. Danach kann das optische Element von proximaler Seite aus in der Fassung angeordnet werden und die Fassung im Instrumentenkörper angeordnet werden. Auf diese Weise kann das optische Element während des Umformens nicht beschädigt werden.

Die oben genannten verschiedenen Ausgestaltungen und Merkmale des Instruments können alle auch Merkmale des hier genannten Verfahrens sein.

Die Erfindung umfasst auch ein optisches Instrument zur Betrachtung eines Lebewesens zu medizinischen Zwecken, dass mit dem vorstehend beschriebenen Verfahren hergestellt wurde.

Das optische Instrument kann dabei alle Merkmale einzeln und in Kombination aufweisen, wie sie vorstehend bereits für ein optisches Instrument beschrieben wurden.

Weitere Aspekte der Erfindung ergeben sich aus der nachfolgenden Beschreibung der bevorzugten Ausführungsbeispiele und den beigefügten Zeichnungen. Es zeigen:
- Figur 1: ein Ausführungsbeispiel eines optischen Instruments in Form eines Videolaryngoskops
- Figur 2: ein Ausführungsbeispiel eines distalen Endes eines Endoskops
- Figur 3: eine schematische Darstellung eines beispielshaften Instruments mit einer Beleuchtungseinheit aus distaler Richtung
- Figur 4: eine schematische Darstellung eines weiteren beispielhaften Instruments mit einer Beleuchtungseinheit aus distaler Richtung
- Figur 5: eine schematische Darstellung eines beispielshaften Instruments mit zwei Beleuchtungseinheiten aus distaler Richtung
- Figur 6: eine schematische Darstellung eines weiteren beispielhaften Instruments mit einer Beleuchtungseinheit aus distaler Richtung
- Figur 7: ein Verfahren zur Herstellung eines optischen Instruments
- Figur 8: das Verfahren aus Figur 7 mit einem weiteren Schritt

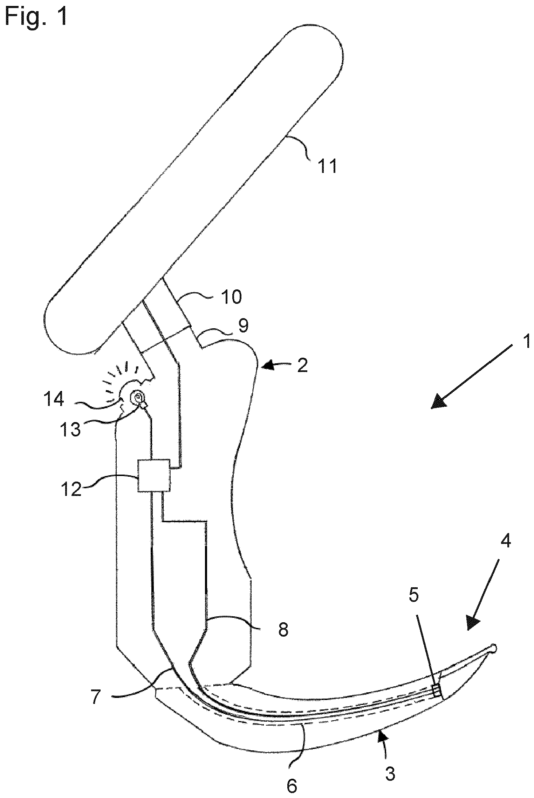

Das in Figur 1 dargestellte optische Instrument ist im vorliegenden Ausführungsbeispiel ein Videolaryngoskop 1 mit einem Handgriff 2 und einem daran befestigten Spatel 3. Der Spatel 3 dient wie zuvor beschrieben der Einführung in den Mund des Patienten zur Betrachtung der Larynx während der Intubation. Zu diesem Zweck ist in einem distalen Abschnitt 4 des Spatels 3 ein Elektronikmodul 5 integriert, das ein Objektiv und einen Bildsensor, sowie eine LED zur Beleuchtung aufweist (nicht dargestellt). Objektiv und Beleuchtung sind nach distal zum Ende des Spatels 3 hin ausgerichtet. In einem Kanal 6 im Spatel 3 verlaufen elektrische Signalleitungen 7 und 8 und verbinden das Elektronikmodul 5 mit einer Steuerung 12 sowie einer Koppelstelle 9 am Handgriff, die mit einem Monitor 11 über einen Stecker 10 verbunden ist. Die Steuerung 12 steuert Bildverarbeitung und Beleuchtung und außerdem eine Signalleuchte 13, die als Statusanzeige 14 dient und dem Anwender den Betriebszustand des Laryngoskops 1 anzeigt.

Die vorderste Linse des Objektivs oder ein Deckglas kann wie vorliegend beschrieben in einer Fassung im Spatel 3 gehalten sein, so dass ein Rand der Fassung nach distal übersteht und in wenigstens einem Abschnitt so verstemmt ist, dass der nicht verstemmte Abschnitt einen Streulichtschutz zwischen der vordersten Linse und der LED bildet.

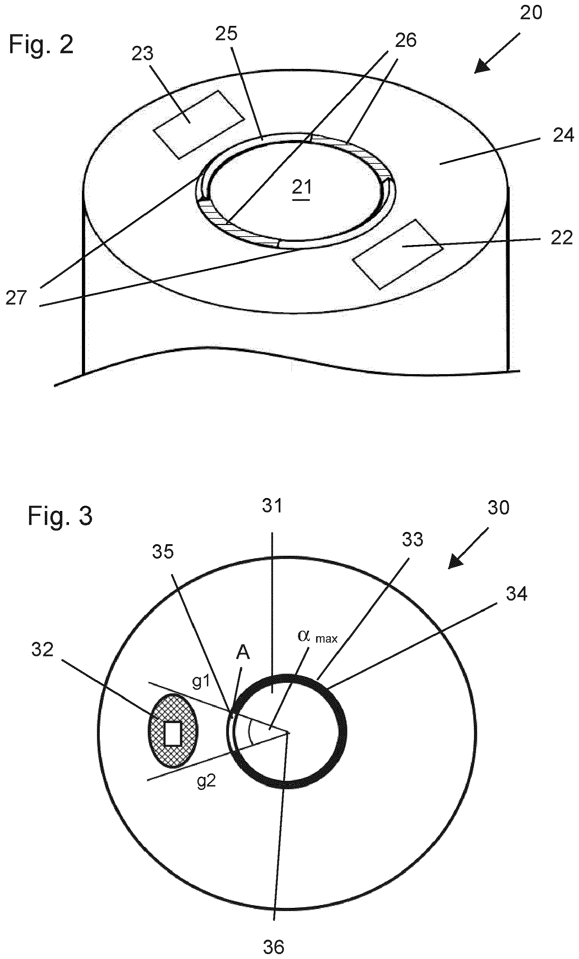

Die Figur 2 zeigt das distale Ende eines beispielhaften Endoskops 20. Dieses weist eine Optik mit einem Deckglas 21 und zwei LEDs 22 und 23 auf, welche zusammen in einer distalen Endfläche 24 des Endoskops 20 angeordnet sind. Die LEDs 22 und 23 sind dabei seitlich und einander gegenüberliegend neben Deckglas 21 montiert, so dass ein beobachteter Bereich gleichmäßig durch die LEDs ausgeleuchtet wird. Die lichtabstrahlende Fläche ist jeweils gleich der Fläche der LED. Optik und Deckglas 21 sind in einer Fassung 25 gehalten, beispielsweise in diese eingeklebt. Die Fassung 25 ist wiederum im Körper des Endoskops 20 montiert. Das distale Ende der Fassung 25 bildet einen über die distale Endfläche 24 hinaus reichenden Rand, mit zwei umgeformten Randabschnitten 26 und zwei nicht umgeformten Randabschnitten 27. Die distale Richtung ist hier oben in der Figur. Die umgeformten Abschnitte 26, hier schraffiert dargestellt, sind verstemmt und halten das Deckglas 21 in distaler Richtung formschlüssig in der Fassung 25. Der distale Rand der Fassung 25 wurde so verstemmt, dass die nicht verstemmten Abschnitte 27 jeweils zwischen dem Deckglas 21 und einer der LEDs 22 und 23 liegen. Auf diese Weise werden das Deckglas 21 und die dahinter befindliche Optik des Instruments 20 vor Beleuchtungslicht und Streulicht aus den LEDs 22 und 23 geschützt. Wie der Figur 2 zu entnehmen ist, ist der Rand in distaler Richtung klein bzw. niedrig im Vergleich zum Durchmesser der Fassung 25 und des Deckglases 21. Es muss nur wenig zusätzliches Material an der Fassung vorgesehen sein, um den distalen Rand auszubilden und das optische Element durch die Fassung auch in distaler Richtung formschlüssig zu sichern.

Im Folgenden sind verschiedene Ausführungsformen eines optischen Instruments schematisch dargestellt. Das Instrument kann wie vorstehend beschrieben ein Endoskop sein, jedoch ebenso ein Laryngoskop, ein Exoskop oder ein anderes optisches Instrument, wie es in der Medizin eingesetzt wird.

Zunächst ist in Figur 3 ein Instrument mit einem Instrumentenkörper 30, einem Deckglas 31 und einer einzelnen LED aus distaler Richtung betrachtet dargestellt. Die LED ist in einer Vertiefung der Endfläche des Instrumentenkörpers 30 neben dem Deckglas 31 angeordnet und dort hinter einem Glasfenster mit einem transparenten Klebstoff vergossen. Der Klebstoff streut das austretende Licht der LED derart, dass die gesamte vergossene Fläche im Betrieb leuchtet. Daher wird vorliegend auf eine lichtabstrahlende Fläche 32 bezuggenommen, die mit der Fläche des Beleuchtungsmittels selbst identisch sein kann, aber wie vorstehend beschrieben auch darüber hinaus reichen kann. Die lichtabstrahlende Fläche 32 ist hier kariert dargestellt und leuchtet ebenso wie die rechteckig in der karierten Fläche dargestellte LED. Das Deckglas 31 ist wiederum in einer Fassung 33 gehalten, beispielsweise mit dieser verklebt. Die Fassung 33 weist einen nach distal vorstehenden Rand auf, welcher in einem Randabschnitt 34 verstemmt wurde, hier dunkel dargestellt. Dieser verstemmte Abschnitt 34 umfasst einen großen Teil des Umfangs der Fassung 33. Der nicht verstemmte Abschnitt 35 des Fassungsrandes, hier hell dargestellt, liegt zwischen der Fläche des Deckglases 31 und der lichtabstrahlenden Fläche 32. Betrachtet man den hier spitzen Winkel, der von zwei gedachten Geraden g1 und g2 eingeschlossen wird, die vom Mittelpunkt 36 des Deckglases 31 ausgehen und die die lichtabstrahlende Fläche 32 schneiden oder berühren, so wird dieser Winkel maximal, wenn die Geraden die Beleuchtungsfläche 32 einschließen und die äußersten Begrenzungen dieser Fläche berühren. Im Bereich dieses Winkels αₘₐₓ liegt der Teil A des Umfangs des distalen Randes der Fassung 33, der nicht umgeformt wurde und den nicht verstemmten Abschnitt 35 bildet. Auf diese Weise ist ein Streulichtschutz definiert, der insbesondere den zentralen Teil der Optik von Streulicht freihält. Ist die lichtabstrahlende Fläche 32 größer als hier dargestellt, wenn sie beispielsweise das Deckglas 31 teilweise umgibt, so kann der Winkel αₘₐₓ natürlich auch erheblich größer sein und der nicht verstemmte Abschnitt 35 einen größeren Teil A betreffen als hier dargestellt. Dies wird von der Größe und Anordnung der lichtabstrahlenden Fläche 32 abhängen.

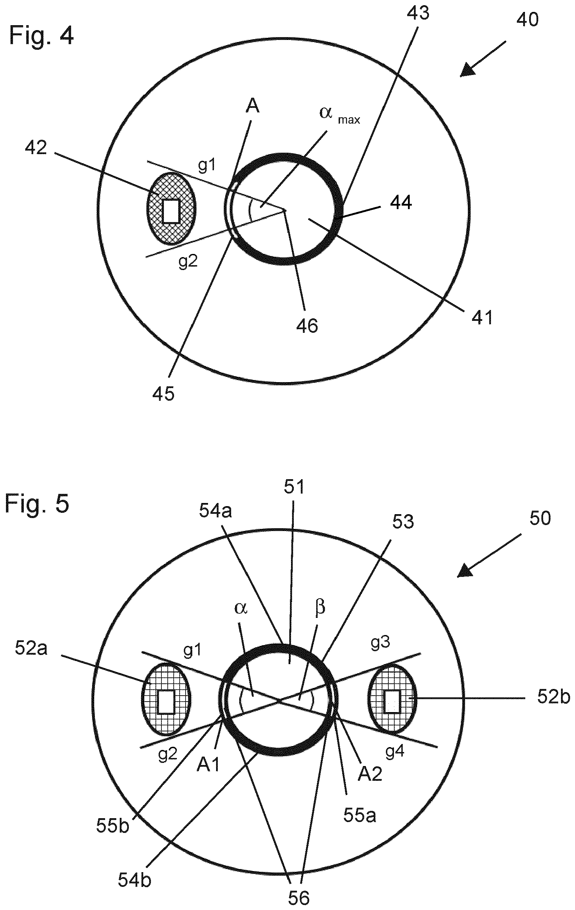

Der Teil A kann als ein Mindestabschnitt der Fassung gesehen werden, der nicht umgeformt wird, um den Streulichtschutz auszubilden. In Figur 4 ist gezeigt, dass der nicht umgeformte Teil auch größer sein kann als der hier definierte Teil A. Es ist wie zuvor ein distales Frontende eines medizinischen Instrumentenkörpers 40 zu sehen, mit einem Deckglas oder einer Frontlinse 41, einer Beleuchtungseinheit in Form einer LED mit einer lichtabstrahlenden Fläche 42, wie zuvor kariert dargestellt, und einer Fassung 43, in der das Deckglas oder die Frontlinse 41 verbaut ist. Die Fassung 43 ist in dem Instrumentenkörper 40 angeordnet. Wiederum sei ein Winkel αₘₐₓ über vom Mittelpunkt 46 des Deckglases oder der Frontlinse 41 ausgehende gedachte Geraden g1 und g2 wie zu Figur 3 ausgeführt definiert. Wie zu erkennen ist, ist der nicht verstemmte Randabschnitt 45 auf dem Umfang der Fassung 43 hier größer als der Teil A, der vom Winkel αₘₐₓ eingeschlossen wird. In dieser Ausgestaltung wurde der nicht verstemmte Abschnitt 45, hier hell dargestellt, zwischen Deckglas 41 und lichtabstrahlender Fläche 42 so gewählt, um einen noch besseren Streulichtschutz für die Optik des Instruments zu bieten. Der Rest des distalen Randes der Fassung 43 ist verstemmt worden (dunkel dargestellt), um das Deckglas 41 formschlüssig in der Fassung zu halten.

Um eine gleichmäßigere Ausleuchtung des beobachteten Bereichs vor dem Instrument zu erreichen, sind häufig zwei oder mehr Beleuchtungseinheiten an dem Instrument vorhanden. Dann ist es notwendig, Beleuchtungs- oder Streulicht von beiden Beleuchtungseinheiten von dem optischen Element fernzuhalten. Bei dem in Figur 5 dargestellten Beispiel sind zwei LEDs mit lichtabstrahlenden Flächen 52a und 52b (kariert dargestellt) seitlich und einander gegenüberliegend neben einer Frontlinse 51 des Instrumentenkörpers 50 angeordnet. Die Frontlinse 51 wird von einer Fassung 53 gehalten, die in dem Instrumentenkörper 50 montiert, beispielsweise eingeklebt ist. Der nach distal vorstehende distale Rand der Fassung 53 weist nun zwei umgeformte, hier verstemmte, Abschnitte 54a und 54b auf (dunkel dargestellt), sowie zwei nicht verstemmte Abschnitte 55a und 55b (hell dargestellt). Die nicht verstemmten Abschnitte sind jeweils zwischen der Frontlinse 51 und den lichtabstrahlenden Flächen 52a bzw. 52b angeordnet. Wie zu Figur 3 beschrieben, liegen die nicht verstemmten Abschnitte 55a und b jeweils in einem Winkelbereich α und β, die durch die Geraden g1 und g2 bzw. g3 und g4 vom Mittelpunkt 56 der Frontlinse 51 ausgehend definiert werden. Die Abschnitte 55a und 55b entsprechen hier genau den Teilen A1 und A2, wie sie durch die Winkel auf dem Umfang der Fassung 53 definiert werden. Es ist natürlich ebenso denkbar, dass die nicht verstemmten Abschnitte 55a und 55b größer sind als A1 oder A2, um die Frontlinse 51 noch mehr von den LEDs abzuschirmen. Die Winkel α und β sind hier als spitze Winkel dargestellt, können jedoch bei entsprechender Ausdehnung der lichtabstrahlenden Flächen 52a und b auch größer als 90° sein. Je nach Ausgestaltung und Anordnung der Beleuchtung können die Winkel und damit die nicht verstemmten Abschnitte der Fassung 53 auch unterschiedlich groß sein. Alle übrigen Abschnitte 54a und b des distalen Randes der Fassung 53 sind im vorliegenden Ausführungsbeispiel verstemmt worden, um die Frontlinse 51 sicher in der Fassung zu halten.

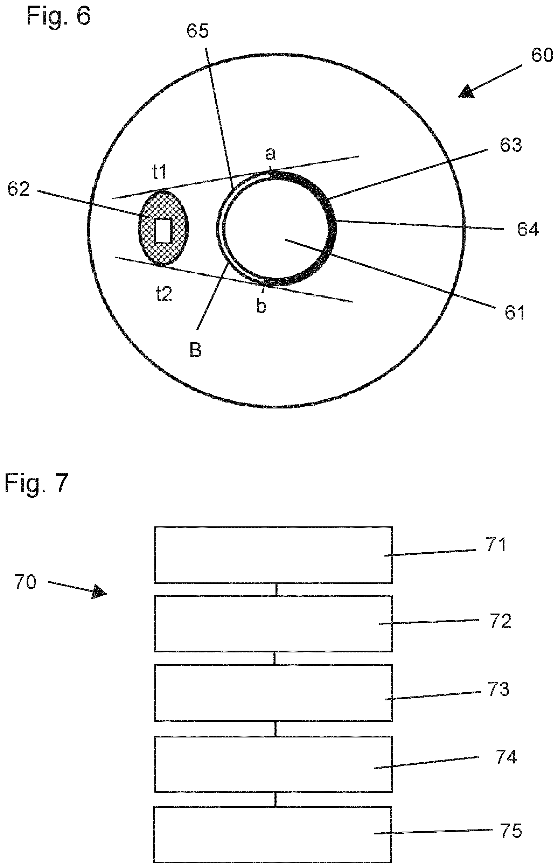

In einem anderen Ausführungsbeispiel, wie es in Figur 6 gezeigt ist, wurde wiederum ein Abschnitt 64 (dunkel dargestellt) einer in dem Instrumentenkörper 60 angeordneten Fassung 63 an deren distalem Rand umgeformt, um ein Deckglas 61 in der Fassung 63 zu halten. Ein dazu komplementärer Abschnitt 65 (hell dargestellt) wurde dagegen nicht umgeformt und umfasst hier einen Teil B vom Umfang des distalen Randes der Fassung 63. Dieser Abschnitt 65 liegt zwischen dem Deckglas 61 und einer LED mit umgebender leuchtender Fläche 62 (kariert dargestellt), die neben dem Deckglas 61 angeordnet ist. Der Teil B wird durch einen Teil des distalen Randes gebildet, der zwischen zwei Punkten a und b liegt, in denen zwei Tangenten t1 und t2 an den Fassungsrand in ihrer Verlängerung die äußersten Abmessungen der lichtabstrahlenden Fläche 62 gerade noch schneiden bzw. berühren. Teil B ist der Abschnitt maximaler Länge, für den dies noch der Fall ist. Abschnitt 65 umfasst hier genau den Teil B, es ist jedoch genauso denkbar, dass der nicht umgeformte Abschnitt größer ist als B und noch mehr Schutz vor in die Optik eintretendem Streulicht bietet.

Auch diese Ausführungsform kann wie zuvor beschrieben mit zwei oder mehr Beleuchtungseinheiten realisiert werden. Besonders bevorzugt umfasst ein nicht umgeformter Abschnitt des distalen Randes der Fassung einen solchen Teil des Umfangs der Fassung, der größer oder gleich dem zuvor definierten Teil A und kleiner oder gleich dem zuvor definierten Teil B ist. Die genaue Auswahl des Abschnitts wird der Fachmann in Abhängigkeit von Lage und Größe der Beleuchtungseinheiten und anderer Gegebenheiten des Instruments treffen.

Ein Verfahren zum Herstellen eines hier beschriebenen optischen Instruments ist in Figur 7 dargestellt. Es umfasst folgende Schritte:
- Bereitstellen 71 eines Instrumentenkörpers, beispielsweise eines Endoskops, Laryngoskops, oder Exos- bzw. Mikroskops,
- Anordnen 72 wenigstens eines optischen Elements zur Aufnahme von Licht aus einem Bereich außerhalb des Instrumentenkörpers und wenigstens einer Beleuchtungseinheit zur Beleuchtung dieses Bereichs in dem Instrumentenkörper, beispielsweise einer Frontlinse und einer LED,
- Anordnen 73 des optischen Elements in einer Fassung, so dass die Fassung sich in distaler Richtung über das optische Element hinaus erstreckt und dort und einen distalen Rand bildet, beispielsweise Einkleben der Frontlinse in die Fassung,
- Befestigen 74 der Fassung in dem Instrumentenkörper, beispielsweise Einkleben der Fassung in einen Spatelteil eines Laryngoskops,
- Umformen 75 des distalen Rands in wenigstens einem Abschnitt, so dass der umgeformte Abschnitt des distalen Randes das optische Element, beispielsweise die Frontlinse, in distaler Richtung formschlüssig hält, und der wenigstens eine nicht umgeformte Abschnitt des distalen Randes so angeordnet ist, dass er einen Streulichtschutz bildet, der aus der Beleuchtungseinheit austretendes Beleuchtungslicht von dem optischen Element fernhält. Das Umformen erfolgt bevorzugt durch Verstemmen des distalen Randes mit einem Stempelwerkzeug, wie dies dem Fachmann bekannt ist.

Es gelten hier die gleichen Überlegungen wie zuvor und die zuvor ausgeführten Ausführungsbeispiele lassen sich durch das Verfahren 70 herstellen.

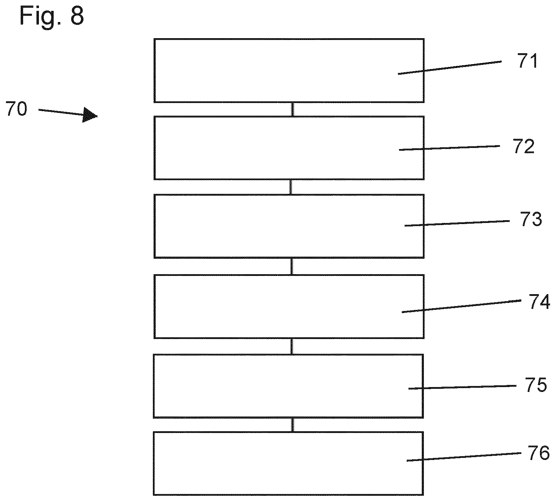

In einer weiteren Variante des Verfahrens 70, dargestellt in Figur 8, umfasst dieses ferner einen Schritt 76, betreffend das Anordnen wenigstens einer zweiten Beleuchtungseinheit in dem Instrumentenkörper und Umformen eines zweiten separaten Abschnitts des distalen Randes, so dass die nicht umgeformten Abschnitte Beleuchtungslicht aus beiden Beleuchtungseinheiten von dem optischen Element fernhalten.

Auch dieses Verfahren ist wie zuvor beschrieben ausführbar und kann die genannten und weitere Ausführungsformen mit zwei oder mehr Beleuchtungseinheiten erzeugen. Insbesondere erfolgt hier wie auch bei der Ausführungsform mit nur einer Beleuchtungseinheit das Umformen des distalen Randes durch Verstemmen mit einem Stempelwerkzeug, wobei der Instrumentenkörper in eine Halterung eingeklemmt und der distale Rand der insbesondere metallenen Fassung durch Applizieren eines Drucks durch das Stempelwerkzeug verformt wird. Beispielsweise wird der vorstehende distale Rand durch das Verstemmen flach gedrückt, so dass er in seiner neuen Breite über das optische Element ragt und dieses so in der Fassung sichert. Zusätzlich kann das Stempelwerkzeug derart ausgebildet sein, dass der flach verstemmte Rand nach außen mit einer Schräge ausgebildet wird. Auf diese Weise wird eine gute Reinigbarkeit gewährleistet.

Ein optisches Instrument, wie es hier beschrieben ist, wurde insbesondere mit dem hier ebenfalls beschriebenen Verfahren hergestellt.

### Bezugszeichenliste:

- 1: Laryngoskop
- 2: Handgriff
- 3: Spatel
- 4: distaler Abschnitt
- 5: Elektronikmodul
- 6: Kanal
- 7: elektrische Signalleitung
- 8: elektrische Signalleitung
- 9: Koppelstelle
- 10: Stecker
- 11: Monitor
- 12: Steuerung
- 13: Signalleuchte
- 14: Statusanzeige
- 20: Endoskopkörper
- 21: Deckglas
- 22: LED
- 23: LED
- 24: distale Endfläche
- 25: Fassung
- 26: verstemmte Randabschnitte
- 27: nicht verstemmte Randabschnitte
- 30: Instrumentenkörper
- 31: Deckglas
- 32: lichtabstrahlende Fläche
- 33: Fassung
- 34: verstemmter Randabschnitt
- 35: nicht verstemmter Randabschnitt
- 36: Mittelpunkt des Deckglases
- g1, g2: Geraden
- 40: Instrumentenkörper
- 41: Deckglas
- 42: lichtabstrahlende Fläche
- 43: Fassung
- 44: verstemmter Randabschnitt
- 45: nicht verstemmter Randabschnitt
- 46: Mittelpunkt des Deckglases
- g1, g2: Geraden
- 50: Instrumentenkörper
- 51: Frontlinse
- 52a/b: lichtabstrahlende Flächen
- 53: Fassung
- 54a/b: verstemmte Abschnitte
- 55a/b: nicht verstemmte Abschnitte
- 56: Mittelpunkt der Frontlinse
- g1, g2, g3, g4: Geraden
- 60: Instrumentenkörper
- 61: Deckglas
- 62: lichtabstrahlende Fläche
- 63: Fassung
- 64: verstemmter Abschnitt
- 65: nicht verstemmter Abschnitt
- a, b: Berührungspunkte der Tangente
- t1, t2: Tangenten
- 70: Verfahren zum Herstellen
- 71: Schritt des Bereitstellens
- 72: Schritt des Anordnens
- 73: weiterer Schritt des Anordnens
- 74: Schritt des Befestigens
- 75: Schritt des Umformens
- 76: weiterer Schritt des Anordnens und Umformens

## Patentansprüche

1. Optisches Instrument zur Betrachtung eines Lebewesens zu medizinischen Zwecken,
mit einem Instrumentenkörper (20, 30, 40, 50, 60),
wobei in dem Instrumentenkörper (20, 30, 40, 50, 60) wenigstens ein optisches Element (21, 31, 41, 51, 61) zur Aufnahme von Licht aus einem Bereich außerhalb des Instrumentenkörpers (20, 30, 40, 50, 60) und wenigstens eine Beleuchtungseinheit (22, 23, 32, 42, 52a, 52b, 62) zur Beleuchtung dieses Bereichs angeordnet sind, wobei das optische Element (21, 31, 41, 51, 61) von einer Fassung (25, 33, 43, 53, 63) in dem Instrument gehalten wird, die sich in distaler Richtung über das optische Element (21, 31, 41, 51, 61) hinaus erstreckt und dort einen distalen Rand bildet, wobei der distale Rand in wenigstens einem Abschnitt so umgeformt ist, dass der umgeformte Abschnitt (26, 34, 44, 54a, 54b, 64) des distalen Randes das optische Element (21, 31, 41, 51, 61) in distaler Richtung formschlüssig hält,
**dadurch gekennzeichnet,**
**dass** wenigstens ein nicht umgeformter Abschnitt (27, 35, 45, 55a, 55b, 65) des distalen Randes so angeordnet ist, dass er einen Streulichtschutz bildet, der aus der Beleuchtungseinheit (22, 23, 32, 42, 52a, 52b, 62) austretendes Beleuchtungslicht von dem optischen Element (21, 31, 41, 51, 61) fernhält.

2. Optisches Instrument nach Anspruch 1, **dadurch gekennzeichnet, dass** die Fassung (25, 33, 43, 53, 63) aus Metall ist.

3. Optisches Instrument nach Anspruch 2, **dadurch gekennzeichnet, dass** der wenigstens eine umgeformte Abschnitt (26, 34, 44, 54a, 54b, 64) mechanisch durch Verstemmen umgeformt wurde.

4. Optisches Instrument nach einem der vorhergehenden Ansprüche, **dadurch gekennzeichnet, dass** die Höhe des distalen Randes des nicht umgeformten Abschnitts (27, 35, 45, 55a, 55b, 65) wenigstens 0,5% und höchstens 5% des Umfangs des distalen Randes beträgt.

5. Optisches Instrument nach einem der vorhergehenden Ansprüche, **dadurch gekennzeichnet, dass** die wenigstens eine Beleuchtungseinheit (22, 23, 32, 42, 52a, 52b, 62) eine lichtabstrahlende Fläche (22, 23, 32, 42, 52a, 52b, 62) aufweist und der wenigstens eine nicht umgeformte Abschnitt (27, 35, 45, 55a, 55b, 65) zwischen dem optischen Element (21, 31, 41, 51, 61) und der lichtabstrahlenden Fläche (22, 23, 32, 42, 52a, 52b, 62) angeordnet ist.

6. Optisches Instrument nach Anspruch 5, wobei der wenigstens eine nicht umgeformte Abschnitt (27, 35, 45, 55a, 55b, 65) des distalen Randes mindestens einen solchen Teil A des Umfangs des distalen Randes umfasst, der einem Winkel αₘₐₓ entspricht, der von Geraden (g1, g2) zwischen dem Mittelpunkt (36, 46, 56) der distalen Fläche des optischen Elements (21, 31, 41, 51, 61) und Punkten der Begrenzung der lichtabstrahlenden Fläche (22, 23, 32, 42, 52a, 52b, 62) derart aufgespannt wird, dass der Winkel maximal wird.

7. Optisches Instrument nach Anspruch 5 oder 6, **dadurch gekennzeichnet, dass** der wenigstens eine nicht umgeformte Abschnitt (27, 35, 45, 55a, 55b, 65) des distalen Randes wenigstens einen Teil B des Umfangs des distalen Randes zwischen zwei Punkten a und b umfasst, derart, dass die jeweilige Tangente (t1, t2), die in diesen Punkten den distalen Rand beführt, die lichtabstrahlende Fläche (22, 23, 32, 42, 52a, 52b, 62) schneidet oder berührt und die Länge des Teils B zwischen a und b maximal wird.

8. Optisches Instrument nach Anspruch 6, **dadurch gekennzeichnet, dass** der wenigstens eine nicht umgeformte Abschnitt (27, 35, 45, 55a, 55b, 65) des distalen Randes mindestens Teil A des Umfangs des distalen Randes umfasst und höchstens einen Teil B des Umfangs zwischen zwei Punkten a und b umfasst, deren jeweilige Tangente (t1, t2), die in diesen Punkten den distalen Rand berührt, die lichtabstrahlende Fläche (22, 23, 32, 42, 52a, 52b, 62) schneidet oder berührt, so dass die Länge des Teils B maximal wird.

9. Optisches Instrument nach einem der vorhergehenden Ansprüche, **dadurch gekennzeichnet, dass** das Instrument ein Laryngoskop (1) mit einem Handgriff (2) und einem Spatel (3) ist, wobei das optische Element (21, 31, 41, 51, 61) und die Beleuchtungseinheit (22, 23, 32, 42, 52a, 52b, 62) am Spatel (3) angeordnet sind.

10. Optisches Instrument nach einem der vorhergehenden Ansprüche, **dadurch gekennzeichnet, dass** das Instrument wenigstens zwei Beleuchtungseinheiten (22, 23, 32, 42, 52a, 52b, 62) aufweist und der distale Rand in wenigstens zwei separaten Abschnitten (26, 34, 44, 54a, 54b, 64) umgeformt ist, so dass die nicht umgeformten Abschnitte (27, 35, 45, 55a, 55b, 65) Beleuchtungslicht aus beiden Beleuchtungseinheiten (22, 23, 32, 42, 52a, 52b, 62) von dem optischen Element (21, 31, 41, 51, 61) fernhalten.

11. Verfahren zum Herstellen (70) eines optischen Instruments zur Betrachtung eines Lebewesens zu medizinischen Zwecken,
mit den Schritten:
Bereitstellen (71)eines Instrumentenkörpers (20, 30, 40, 50, 60),
Anordnen (72) wenigstens eines optischen Elements (21, 31, 41, 51, 61) zur Aufnahme von Licht aus einem Bereich außerhalb des Instrumentenkörpers (20, 30, 40, 50, 60) und wenigstens einer Beleuchtungseinheit (22, 23, 32, 42, 52a, 52b, 62) zur Beleuchtung dieses Bereichs in dem Instrumentenkörper (20, 30, 40, 50, 60),
Anordnen (73) des optischen Elements (21, 31, 41, 51, 61) in einer Fassung (25, 33, 43, 53, 63), so dass die Fassung (25, 33, 43, 53, 63) sich in distaler Richtung über das optische Element (21, 31, 41, 51, 61) hinaus erstreckt und dort und einen distalen Rand bildet,
Befestigen (74) der Fassung (25, 33, 43, 53, 63) in dem Instrumentenkörper (20, 30, 40, 50, 60),
**gekennzeichnet durch** den Schritt des
Umformens (75) des distalen Rands in wenigstens einem Abschnitt, so dass der umgeformte Abschnitt (26, 34, 44, 54a, 54b, 64) des distalen Randes das optische Element (21, 31, 41, 51, 61) in distaler Richtung formschlüssig hält, und der wenigstens eine nicht umgeformte Abschnitt (27, 35, 45, 55a, 55b, 65) des distalen Randes so angeordnet ist, dass er einen Streulichtschutz bildet, der aus der Beleuchtungseinheit (22, 23, 32, 42, 52a, 52b, 62) austretendes Beleuchtungslicht von dem optischen Element (21, 31, 41, 51, 61) fernhält.

12. Verfahren nach Anspruch 11, **gekennzeichnet durch** den weiteren Schritt (76): Anordnen wenigstens einer zweiten Beleuchtungseinheit (22, 23, 32, 42, 52a, 52b, 62) in dem Instrumentenkörper (20, 30, 40, 50, 60) und Umformen eines zweiten separaten Abschnitts (26, 34, 44, 54a, 54b, 64) des distalen Randes, so dass die nicht umgeformten Abschnitte (27, 35, 45, 55a, 55b, 65) des distalen Randes Beleuchtungslicht aus beiden Beleuchtungseinheiten (22, 23, 32, 42, 52a, 52b, 62) von dem optischen Element (21, 31, 41, 51, 61) fernhalten.

13. Verfahren nach Anspruch 11 oder 12, wobei das Umformen des distalen Randes in dem wenigstens einen Abschnitt durch Verstemmen des distalen Randes erfolgt.
